(19) Europäisches Patentamt
European Patent Office
Office européen des brevets

(11) Numéro de publication : **0 193 471 B2**

# (12) NOUVEAU FASCICULE DE BREVET EUROPEEN

(45) Date de publication du nouveau fascicule du brevet : **06.10.93 Bulletin 93/40**

(51) Int. Cl.[5] : **A61K 7/48,** A61K 7/135, A61K 33/40

(21) Numéro de dépôt : **86400415.5**

(22) Date de dépôt : **26.02.86**

## (54) Utilisation dans les domaines thérapeutique et cosmétique d'une solution anhydre de peroxyde d'hydrogène.

(30) Priorité : **26.02.85 LU 85789**

(43) Date de publication de la demande : **03.09.86 Bulletin 86/36**

(45) Mention de la délivrance du brevet : **11.10.89 Bulletin 89/41**

(45) Mention de la décision concernant l'opposition : **06.10.93 Bulletin 93/40**

(84) Etats contractants désignés : **AT BE CH DE FR GB IT LI NL**

(56) Documents cités :
**EP-A- 0 121 660**
**EP-A- 0 141 759**
**DE-A- 3 334 854**
**DE-A- 3 431 755**
**DE-B- 1 262 982**
**DE-B- 2 025 237**
**FR-A- 2 145 400**
**FR-A- 2 515 034**
**GB-A- 545 288**
**GB-A- 632 084**
**US-A- 4 140 772**

(56) Documents cités :
**W.MACHU, "Das Wasserstoffperoxid", 2ème.édition, Springer Verlag, Vienne (1951), pages 201 et 202.**
**HUGO JANISTYN; "Handbuch der Kosmetika und Riechstoffe", vol.III, 2ème édition, Heildelberg (DE) (1973), pp. 659-660**
**RÖMPPS Chemie Lexicon, 5ème édition (1962), pp.5599-5605**
**J.SCHNEIDER, Seifen-Öle-Fette-Wachse 95/18 (1969), page 629**

(73) Titulaire : **L'OREAL**
**14, Rue Royale**
**F-75008 Paris (FR)**

(72) Inventeur : **Jacquet, Bernard**
**50, rue Prosper Legouté**
**F-92160 Antony (FR)**
Inventeur : **Gaetani, Quintino**
**64, Avenue Hoche**
**F-93270 Sevran (FR)**
Inventeur : **Hocquaux, Michel**
**70, rue du Rendez-Vous**
**F-75012 Paris (FR)**

(74) Mandataire : **Nony, Michel et al**
**Cabinet NONY & CIE 29, rue Cambacérès**
**F-75008 Paris (FR)**

**EP 0 193 471 B2**

## Description

La présente invention a pour objet l'utilisation d'une solution anhydre de peroxyde d'hydrogène dans un solvant organique dans les domaines thérapeutique et cosmétique.

Le peroxyde d'hydrogène en solution aqueuse, ou eau oxygénée, est bien connu pour ses propriétés bactéricides et oxydantes. L'eau oxygénée est tout particulièrement recommandée pour la désinfection cutanée ou buccale, la désinfection des plaies et des mains, et dans le traitement des mycoses, des inflammations et des ulcères. L'eau oxygénée trouve tout particulièrement une application dans le traitement de l'acné et d'autres maladies de la peau en inhibant la croissance des principales souches de bactéries responsables des manifestations acnéiques.

Les propriétés oxydantes de l'eau oxygénée sont recherchées en cosmétique essentiellement dans les traitements de décoloration des cheveux ou dans les teintures dites d'oxydation mais également dans les opérations de permanente, comme agent de neutralisation ou de fixation.

Les solutions d'eau oxygénée généralement utilisées sont à 6 ou 9 % (20 ou 30 volumes) mais l'on peut également utiliser dans certaines conditions des solutions à concentration plus élevée comme par exemple à 30 % (100 volumes).

Les solutions diluées de peroxyde d'hydrogène présentent cependant l'inconvénient de manquer de stabilité dans le temps bien qu'elles puissent être améliorées par l'addition d'agents stabilisants. Dans les traitements topiques sus-mentionnés et particulièrement dans le traitement de l'acné, on a noté une mauvaise pénétration du peroxyde d'hydrogène et l'apparition d'un effet prononcé de dessèchement de la peau. Il peut d'autre part éventuellement se produire certaines lésions tissulaires si la peau est exposée à une quantité excessive de peroxyde d'hydrogène.

En vue de remédier à ces différents inconvénients, notamment à l'absence de stabilité de l'eau oxygénée, et de façon à assurer une meilleure disponibilité du peroxyde d'hydrogène, la présente invention propose l'utilisation de solutions essentiellement anhydres de peroxyde d'hydrogène dans les domaines thérapeutique et cosmétique.

L'état de la technique concernant des solutions à base de peroxyde d'hydrogène dans des solvants organiques est essentiellement représenté par les documents suivants:

GB-A-632 084 décrit des solutions liquides antiseptiques de peroxyde d'urée résultant de l'association de peroxyde d'hydrogène et d'urée dans un alcool polyhydrique.

DE-A-1 262 982 décrit des solutions de peroxyde d'hydrogène dans des di ou polyesters d'acides dicarboxyliques insaturés, utilisables comme catalyseurs de réaction de polymérisation.

FR-A-2 145 400 a pour objet des solutions d'un composé peroxydé tel que le peroxyde d'hydrogène dans des esters d'alcoyle ou dans des acides organiques, ces solutions étant employées dans le blanchiment des fibres textiles.

EP-A-4 121 660 concerne un procédé de préparation de solutions de peroxyde d'hydrogène dans divers solvants organiques utilisables pour des réactions d'oxydation ou d'époxydation.

Les solutions anhydres de peroxyde d'hydrogène, selon l'invention, peuvent être utilisées indifféremment soit dans le domaine thérapeutique notamment dans le traitement de l'acné, soit dans le domaine cosmétique, plus particulièrement capillaire, dans les opérations de décoloration, de teinture d'oxydation et de neutralisation d'une permanente, ou d'un défrisage des cheveux.

L'invention a donc pour objet l'utilisation d'une solution liquide, essentiellement anhydre de peroxyde d'hydrogène, dans au moins un solvant organique, dans les domaines thérapeutique et cosmétique, ladite solution contenant de 0,1 + 20 % en poids de peroxyde d'hydrogène et moins de 1 % en poids d'eau.

Selon une forme préférée, la solution a une teneur en peroxyde d'hydrogène comprise entre 1 et 10 % en poids et une teneur en eau inférieure à 0,5 %.

Le solvant organique dans lequel est dissous le peroxyde d'hydrogène peut être de nature très variée mais l'on préfère, selon l'invention, utiliser les solvants organiques suivants qui sont thérapeutiquement et cosmétiquement acceptables :

1) les alcools ayant de 2 à 20 atomes de carbone tels que l'éthanol, le n-propanol, l'isopropanol, le cyclohexanol, l'alcool amylique, l'alcool oléique et l'alcool benzylique,

2) les polyols tels que les glycols et plus particulièrement l'éthylène glycol, le propylène glycol, le diéthylène glycol, le glycérol, et leurs éthers, et

3) les polyéthers oligomères tels que ceux de l'oxyde d'éthylène, de l'oxyde de propylène et leurs éthers, ainsi que les polyéthers oligomères répondant à la formule suivante :

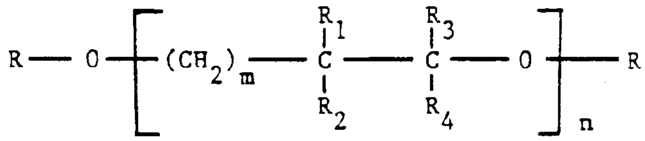 (I)

dans laquelle :

R représente un radical alkyle, linéaire ou ramifié, ayant de 1 à 12 atomes de carbone,

$R_1$, $R_2$, $R_3$ et $R_4$, identiques ou différents, représentent un atome d'hydrogène ou un radical alkyle ayant de 1 à 6 atomes de carbone, au moins deux des radicaux $R_1$, $R_2$, $R_3$ ou $R_4$ représentant un atome d'hydrogène,

m est 1 à 4,

et n est une valeur moyenne supérieure ou égale à 2 et de préférence comprise entre 4 et 50,

le nombre d'atomes de carbone dans chaque unité répétitive, identique ou différente, étant au moins égal à 4.

La masse moléculaire moyenne en nombre de ces polyéthers oligomères est généralement comprise entre 200 et 5 000 et leur viscosité entre 0,002 et 1 Pa·s mesurée à 25 °C et de préférence entre 0,01 et 0,1 Pa · s.

Parmi les polyéthers oligomères représentés par la formule ci-dessus, celui ayant donné d'excellents résultats tant dans le domaine thérapeutique que cosmétique est le diméthyléther du polytétrahydrofuranne répondant à la formule suivante :

$$CH_3 - O \left[ (CH_2)_2 - CH_2 - CH_2 - O \right]_n CH_3 \qquad (II)$$

dans laquelle n est une valeur moyenne comprise entre 4 et 10.

Il va de soi que le solvant organique doit être stable en présence du peroxyde d'hydrogène, ce solvant étant choisi en fonction de l'utilisation à laquelle est destinée la solution anhydre.

Les procédés de préparation des solutions anhydres de peroxyde d'hydrogène sont connus et ont été abondamment décrits dans la littérature.

Pour la préparation des solutions mises en oeuvre selon l'invention deux procédés différents peuvent être envisagés selon la nature du solvant organique.

Lorsque le solvant organique n'est pas miscible à l'eau, le peroxyde d'hydrogène est extrait, sous forte agitation, à partir d'une solution aqueuse à 60 % d'eau oxygénée (200 volumes).

Après agitation entre 3 à 8 heures, on sature la phase aqueuse par du chlorure de sodium et l'on décante la phase organique qui est ensuite séchée sur sulfate de sodium.

Lorsque le solvant organique est miscible à l'eau, l'eau est éliminée par distillation azéotropique. Dans ce cas, l'on utilise alors, en mélange avec le solvant organique, un co-solvant présentant la propriété de former un mélange azéotrope avec l'eau.

Parmi les solvants miscibles à l'eau qui peuvent être utilisés pour la préparation des solutions anhydres de peroxyde d'hydrogène on peut notamment mentionner parmi les alcools, l'éthanol, le n-propanol, l'isopropanol et le cyclohexanol; parmi les polyols, l'éthylèneglycol, le propylèneglycol et le glycérol ; et parmi les polyéthers oligomères, ceux d'oxyde d'éthylène, d'oxyde de propylène et leurs éthers.

Dans ce cas, l'on utilise comme co-solvant susceptible de former un mélange azéotropique avec l'eau, l'acétonitrile, le tertio-butanol, le cyclohexane, l'heptane, le pentane, le dichloro-1,2 éthane ou l'acétate d'éthyle.

L'entraînement azéotropique de l'eau se fait à température d'ébullition du mélange. Après refroidissement on sèche sur sulfate de sodium anhydre.

Le dosage du peroxyde d'hydrogène dans le solvant organique est ensuite déterminé par polarographie.

En fonction de la nature du solvant, la teneur en peroxyde d'hydrogène des solutions peut varier dans de larges limites.

Ainsi, à l'aide du diméthyléther du polytétrahydrofuranne de viscosité 0,022 Pa · s, il est possible d'extraire jusqu'à 15 % de peroxyde d'hydrogène alors qu'avec l'alcool amylique, la teneur en peroxyde d'hydrogène est de l'ordre de 7,6 %.

Bien entendu, les solutions anhydres de peroxyde d'hydrogène peuvent être obtenues à partir d'une eau oxygénée plus concentrée mais pour des raisons de commodité et surtout de sécurité on préfère utiliser une solution à 60 %.

Comme procédé par distillation azéotropique on peut en particulier faire référence à la demande de brevet européen n° 0 121 660 publiée le 17 octobre 1984.

Les solutions anhydres de peroxyde d'hydrogène trouvent tout particulièrement une application dans le traitement de l'acné et d'autres dermatoses, des ulcères et pour le blanchiment et la désinfection de la peau.

Dans ce type d'application, le solvant organique doit être choisi parmi ceux permettant une bonne pénétration et ne provoquant aucune irritation sur la peau.

Parmi les solvants organiques qui conviennent pour cette application on peut citer: le n-propanol, l'isopropanol, l'alcool benzylique, les polyéthylène glycols, les polyéthers du tétrahydrofuranne, l'éther monoéthylique du diéthylène glycol, l'éther monoéthylique du propylène glycol et le propylène glycol.

Comme solvant particulièrement préféré on peut citer le diméthyléther du polytétrahydrofuranne dont une solution à 3 % en peroxyde d'hydrogène conduit à des propriétés comédolytiques équivalentes à celles d'une solution à 6 % de peroxyde de benzoyle dans le test de la souris rhino, alors qu'une teneur équivalente en peroxyde d'hydrogène en milieu aqueux ne conduit à aucune activité.

Si la teneur en peroxyde d'hydrogène de la solution anhydre s'avérait trop élevée pour le type de traitement envisagé, il est possible de la diluer à la teneur voulue à l'aide soit du solvant d'extraction soit de tout autre solvant miscible en vue de conduire à une solution homogène acceptable.

Les solutions anhydres de peroxydes d'hydrogène peuvent également contenir d'autres ingrédients notamment des produits permettant d'augmenter la viscosité ceci en vue d'éviter tout écoulement de la solution lors de l'application.

Dans le domaine capillaire, les solutions anhydres de peroxyde d'hydrogène peuvent servir à la décoloration des cheveux en vue de leur donner un aspect plus clair, elles servent également d'agent oxydant dans la teinture dite d'oxydation ou dans la deuxième phase dite de repontage d'une déformation permanente.

Dans ce type d'application, les compositions se présentent préférentiellement sous forme de solutions plus ou moins épaissies.

Elles peuvent contenir divers adjuvants utilisés habituellement en cosmétique comme des tensio-actifs, par exemple non ioniques ou anioniques, des épaississants, des tiers solvants comme l'éthanol, des éthers de glycols, etc...

Au moment de l'emploi ces compositions anhydres peuvent être mélangées à des solutions ammoniacales, elles-mêmes renfermant éventuellement des tensio-actifs, des épaississants, des séquestrants, des tiers solvants.

Dans le cas des opérations de décoloration et de teinture le temps de pose des compositions selon l'invention se situe entre 5 et 45 minutes.

Dans le cas du deuxième temps d'une permanente ou d'un défrisage, le temps de pose variera entre 5 et 20 minutes.

Il a été observé qu'avec les compositions anhydres selon l'invention, on obtenait, dans le cas d'une décoloration, un éclaircissement plus fort qu'avec des solutions aqueuses de peroxyde d'hydrogène de même titre.

La présente invention a également pour objet une solution essentiellement anhydre de peroxyde d'hydrogène dans au moins un solvant organique, ledit solvant étant essentiellement constitué par un polyéther oligomère de formule (I) et de préférence le diméthyléther du polytétrahydrofuranne de formule (II) ci-dessus, ladite solution contenant de 0,1 à 20 % en poids de peroxyde d'hydrogène et moins de 1 % en poids d'eau.

De préférence la solution essentiellement anhydre à base de polyéther oligoméré a une teneur en peroxyde d'hydrogène comprise entre 1 et 10 % en poids et une teneur en eau inférieure à 0,5 %.

On va maintenant donner à titre d'illustration plusieurs exemples de préparation des solutions anhydres de peroxyde d'hydrogène ainsi que plusieurs exemples d'utilisation dans les domaines thérapeutique et cosmétique.

Exemple 1

Solution de peroxyde d'hydrogène dans le diméthyléther du polytétrahydrofuranne.

A une solution agitée de 500 ml de diméthyléther du polytétrahydrofuranne ayant une viscosité de 0,022 Pa · s, on ajoute lentement en refroidissant 500 ml d'une solution d'eau oxygénée à 60 % (200 volumes).

Le mélange hétérogène est maintenu sous bonne agitation pendant 3 hl/2. On additionne 50 g de chlorure de sodium et l'on maintient l'agitation une demi-heure.

La phase organique est alors décantée puis séchée sur sulfate de sodium anhydre et enfin filtrée.

La teneur en peroxyde d'hydrogène ($H_2O_2$) est déterminée par polarographie: teneur 15 %.

Une étude de stabilité sur 4 mois n'a pas permis de montrer une variation de la teneur en peroxyde d'hydrogène de la solution.

Le diméthyléther du polytétrahydrofuranne de viscosité 0,022 Pa·s est obtenu selon le procédé suivant:

Dans un réacteur de 20 litres muni d'une agitation, d'un réfrigérant, d'un thermomètre et d'une arrivée d'azote, on introduit 5,32 kg de tétrahydrofuranne distillé et 5,8 kg d'orthoformiate de méthyle également distillés.

Le mélange est alors agité sous azote et refroidi à 13°C.

On ajoute ensuite 120 ml d'anhydride trifluorométhane sulfonique pour initier la polymérisation. Après 4 heures à 18-20°C, on désactive le catalyseur par 120 g de soude pure en solution dans 600 ml d'eau permutée. Les produits volatils sont alors chassés sous vide à 80°C. On introduit ensuite après refroidissement 6 litres de cyclohexane distillée et 120 g de noir de carbone en poudre. On agite une heure puis filtre le mélange en rinçant le précipité avec du cyclohexane distillé.

Le filtrat est ensuite agité sous vide à 50°C puis à 80°C et enfin à 100°C pour éliminer le solvant ainsi que les fractions de polyéthers volatils. On récupère ainsi 3,75 kg d'un composé huileux et incolore.

L'huile obtenue présente une viscosité dynamique à 25 °C de 0,022 Pa·s et se fige à -1°C.

Exemple 2

Solution de peroxyde d'hydrogène dans l'alcool amylique.

A une solution agitée de 500 ml d'alcool amylique, on ajoute lentement en refroidissant 500 ml d'une solution d'eau oxygénée à 60 % (200 volumes).

Le mélange hétérogène est maintenu sous bonne agitation pendant 4 heures. On additionne 50 g de chlorure de sodium et l'on maintient l'agitation une heure.

La phase organique est ensuite décantée puis séchée sur sulfate de sodium et filtrée. La teneur en peroxyde d'hydrogène est déterminée par polarographie.

Teneur: 7,6 %.

Exemple 3

Solution de peroxyde d'hydrogène dans l'alcool oléique.

Selon le même mode opératoire que celui décrit à l'exemple 2, l'extraction par l'alcool oléique conduit à une solution anhydre de peroxyde d'hydrogène ayant une teneur de: 4,5 %.

Exemple 4

Solution de peroxyde d'hydrogène dans le polyéthylène glycol (PEG 200).

A 400 ml d'éther éthylique on ajoute sous agitation à 20 °C 400 ml d'eau oxygénée à 60 % (200 volumes).

Après avoir poursuivi l'agitation pendant environ 1 heure on procède alors à l'addition de chlorure de sodium jusqu'à saturation puis l'on décante la phase éthérée que l'on sèche ensuite sur sulfate de sodium anhydre.

Après filtration on ajoute à la solution de peroxyde d'hydrogène dans l'éther, 100 ml de polyéthylène glycol (PEG 200)® à une température de 20 °C. Après agitation on évapore l'éther et obtient une solution limpide anhydre de peroxyde d'hydrogène dans le polyéthylène glycol ayant une teneur de : 12,5 %.

Exemple 5

Solution de peroxyde d'hydrogène dans le méthoxypropanol.

Comme décrit ci-dessus à l'exemple 4 on prépare une solution de peroxyde d'hydrogène dans l'éther éthylique à partir de 200 ml d'éther et 200 ml d'eau oxygénée à 60 %.

A cette solution on ajoute à 20 °C sous agitation 100 ml de méthoxypropanol ("DOWANOL PM"® de la Société DOW CHEMICAL).

Après évaporation de l'éther on obtient une solution anhydre de peroxyde d'hydrogène dans le méthoxypropanol ayant une teneur de 4,9 %.

Compositions anti-acnéiques

Exemple A

| | |
|---|---|
| Solution anhydre à 15 % de peroxyde d'hydrogène dans le diméthyléther du polytétrahydrofuranne (selon l'exemple 1) | 20 g |
| diméthyléther du polytétrahydrofuranne de viscosité 0,022 Pa · s | 80 g |

La solution ainsi obtenue a une teneur de 3 % en peroxyde d'hydrogène possédant des propriétés comédolytiques équivalentes à celles d'une solution à 6 % de peroxyde de benzoyle.

Exemple B

| | |
|---|---|
| Solution anhydre de peroxyde d'hydrogène à 15 % dans le diméthyléther du polytétrahydrofuranne (selon l'exemple 1) | 25 g |
| éther monoéthylique du diéthylèneglycol | 75 g |

La solution anhydre obtenue a une teneur de 3,75 % en peroxyde d'hydrogène.

Exemple C

| | |
|---|---|
| Solution anhydre de peroxyde d'hydrogène à 15 % dans le diméthyléther du polytétrahydrofuranne (selon l'exemple 1) | 25 g |
| Alcool n-propylique | 36 g |
| Alcool isopropylique | 36 g |
| Hydroxypropylcellulose | 3 g |

Ce gel anhydre présente une bonne activité comédolytique comme ceci a été confirmé dans le test de la souris rhino.

Exemple D

| | |
|---|---|
| Solution anhydre de peroxyde d'hydrogène à 15 % dans le diméthyléther du polytétrahydrofuranne (selon l'exemple 1) | 40 g |
| Alcool n-propylique | 40 g |
| Propylène glycol | 18 g |
| Hydroxypropylcellulose | 2 g |

Exemples E à K

(Voir Tableau page 7)

**Lotions anti-acnéiques**

| EXEMPLE | E | F | G | H | I | J | K |
|---|---|---|---|---|---|---|---|
| - Solution à 12,9% $H_2O_2$ dans diméthyl éther du polytétrahydrofuranne | 24 | - | 24 | - | | 24 | 24 |
| - Solution à 12,5% $H_2O_2$ dans polyéthylène glycol | - | 24 | | 24 | - | - | - |
| - Solution à 4,9% $H_2O_2$ dans méthoxypropanol | - | - | | - | 63 | - | - |
| - Sulfate d'o-oxy-quinoléine | 0,02 | 0,02 | 0,02 | 0,02 | 0,02 | 0,02 | 0,02 |
| - Phénacétine | 0,05 | 0,05 | 0,05 | 0,05 | 0,05 | 0,05 | 0,05 |
| - Acide citrique | 0,2 | 0,2 | 0,2 | 0,2 | 0,2 | 0,2 | 0,2 |
| - Acide salicylique | 0,2 | 0,2 | 0,2 | 0,2 | 0,2 | 0,2 | 0,2 |
| - Alcool absolu | 60,4 | | - | - | - | 60,4 | - |
| - Isopropanol | - | 60,4 | 60,4 | 60,4 | | | 37,7 |
| - méthoxypropanol | - | - | - | - | 21,43 | | - |
| - Glycérides oléiques polyoxyéthylénés ("LABRAFIL M 1944CS"®) | - | 15,13 | - | - | 15,1 | 15,13 | - |
| - Glycérides en $C_8/C_{10}$ polyoxyéthylénés ("LABRASOL"®) | - | - | 15,13 | - | - | - | - |
| - Triglycérides d'acide gras ("Miglyol 812"®) | 15,13 | - | - | 15,13 | - | - | - |
| - Silicone volatile | - | | - | - | - | - | 37,83 |

Exemple L

Exemple de décoloration

Composition 1

| | |
|---|---|
| Solution anhydre de peroxyde d'hydrogène à 8,8 % dans le diméthyléther du polytétrahydrofuranne | 68 g |
| nonyl phénol à 9 moles d'oxyde d'éthylène | 27 g |
| alcool éthylique | 5 g |
| | 100 g |

Composition 2

ammoniaque 22° Baumé 100 g

On mélange au moment de l'emploi dans les proportions suivantes: 90 g de la composition 1 avec 10 g de la composition 2.

On obtient un mélange homogène et liquide que l'on fait poser 30 minutes sur une chevelure châtain.

Après rinçage et séchage, on constate une forte décoloration.

Exemple M

Exemple de décoloration

Composition 1

| | |
|---|---|
| Solution anhydre de peroxyde d'hydrogène à 9,8 % dans le diméthyléther du polytétrahydrofuranne | 61 g |
| « Texapon WW99 »® (Lauryl sulfate de monoisopropanolamine vendu par HENKEL) | 10 g |
| alcool éthylique | 29 g |
| | 100 g |

Composition 2

| | |
|---|---|
| alcool oléique glycérolé à 2 moles de glycérol | 5 g |
| alcool oléique glycérolé à 4 moles de glycérol | 5 g |
| acide oléique | 5 g |
| diéthanolamine oléique | 5 g |
| diéthanol amide oléique | 12 g |
| alcool éthylique | 10 g |
| éthyl glycol | 12 g |
| EDTA | 0,2 g |
| ammoniaque 22° Baumé | 11 g |
| eau q.s.p. | 100 g |

On mélange au moment de l'emploi 2 parties de la composition 1 pour une partie de la composition 2.

On obtient un mélange homogène que l'on fait poser 30 minutes sur une chevelure châtain.

Après rinçage et séchage, on constate un éclaircissement moyen.

Exemple N

Exemple de coloration.

Composition 1

| | |
|---|---|
| paraphénylènediamine | 0,185 g |
| paraaminophénol | 0,555 g |
| 1-méthyl 2-hydroxy 4-β hydroxy éthylamine benzène | 0,308 g |
| dichlorhydrate de 2-4 diaminophénoxy éthanol | 0,123 g |
| éthanol | 20,4 g |
| thiolactate d'ammonium (à 50 % en équivalent d'acide thiolactique) | 17,9 g |
| ammoniaque à 20 % de $NH_3$ | 6,16 g |
| eau déminéralisée qsq | 100 g |

Composition 2

| | |
|---|---|
| solution anhydre de peroxyde d'hydrogène à 15,8 % dans le diméthyl éther du polytétrahydrofuranne | 100 g |

Au moment de l'emploi on mélange 81 g de la composition 1 avec 19 g de la composition 2.

Le mélange obtenu est alors appliqué sur des cheveux naturels à 90 % de cheveux blancs. Après 30 minutes de pose, on rince les cheveux, on les lave à l'aide d'un shampooing et les sèche.

La coloration des cheveux obtenue est châtain clair tirant sur l'acajou.

Exemple O

Exemple de déformation permanente des cheveux.

On applique sur les cheveux la composition réductrice suivante :

| | |
|---|---|
| acide thioglycolique | 6 g |
| ammoniaque qsq pH 9,5 | |
| agent séquestrant | 0,2 g |
| alcool gras polyoxyéthyléné à 20 moles d'oxyde d'éthylène | 1 g |
| Parfum | 0,5 g |
| eau qsq | 100 g |

Les cheveux une fois imprégnés on les enroule sur des bigoudis puis on laisse agir la composition pendant 10 à 15 minutes.

Après rinçage, on applique la solution oxydante suivante:

| | |
|---|---|
| solution anhydre de peroxyde d'hydrogène à 9,8 % dans le diméthyléther® de polytétrahydrofuranne | 61 g |
| Texapon WW 99 (laurylsulfate de monoisopropanolamine vendu par HENKEL) | 10 g |
| Alcool éthylique | 29 g |

On laisse agir cette composition pendant 5 minutes puis on rince les cheveux de l'eau et enlève les bigoudis.

On obtient ainsi une excellente permanente, les cheveux étant doux au toucher et nerveux.

## Revendications

1. Utilisation d'une solution liquide essentiellement anhydre de peroxyde d'hydrogène, dans au moins un solvant organique, pour la préparation d'une composition thérapeutique destinée au traitement de l'acné, des dermatoses et des ulcères de la peau, ledit solvant organique étant choisi parmi un alcool ayant de 2 a 20 atomes de carbone, un polyol et un polyéther oligomère et ladite solution contenant de 0,1 à 20 % en poids de peroxyde d'hydrogène, comme seul principe actif, et moins de 1 % en poids d'eau.

2. Utilisation d'une solution liquide essentiellement anhydre de peroxyde d'hydrogène, dans au moins un solvant organique, dans les opérations de décoloration ou de teinture d'oxydation des cheveux et de neutralisation de permanente ou de défrisage des cheveux, ledit solvant organique étant choisi parmi un alcool ayant de 2 à 20 atomes de carbone, un polyol et un polyéther oligomère et ladite solution contenant de 0,1 à 20 % en poids de peroxyde d'hydrogène, comme seul principe actif, et moins de 1 % en poids d'eau.

3. Utilisation, selon l'une des revendications 1 ou 2, caractérisée par le fait que la solution essentiellement anhydre a une teneur en peroxyde d'hydrogène comprise entre 1 et 10 % en poids et une teneur en eau inférieure à 0,5 %.

4. Utilisation selon l'une des revendications 1 ou 2, caractérisée par le fait que l'alcool ayant de 2 à 20 atomes de carbone est choisi dans le groupe constitué par l'éthanol, le n-propanol, l'alcool amylique, l'isopropanol,

l'alcool oléique, le cyclohexanol et l'alcool benzylique.

**5.** Utilisation selon l'une des revendications 1 ou 2, caractérisée par le fait que le polyol est choisi dans le groupe constitué par l'éthylène glycol, le propylène glycol, le diéthylène glycol, le glycérol et leurs éthers.

**6.** Utilisation selon l'une des revendications 1 ou 2, caractérisée par le fait que le polyéther oligomère est choisi parmi ceux de l'oxyde d'éthylène, de l'oxyde de propylène et leurs éthers ainsi que parmi les polyéthers oligomères répondant à la formule suivante :

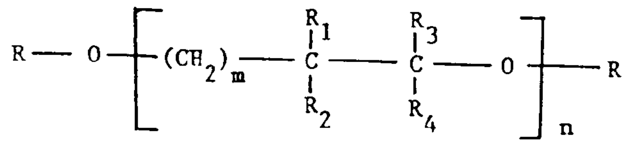

dans laquelle :

R représente un radical alkyle, linéaire ou ramifié, ayant de 1 à 12 atomes de carbone,

$R_1$, $R_2$, $R_3$ et $R_4$, identiques ou différents, représentent un atome d'hydrogène ou un radical alkyle ayant de 1 à 6 atomes de carbone, au moins deux des radicaux $R_1$, $R_2$, $R_3$ ou $R_4$ représentant un atome d'hydrogène,

m est 1 à 4,

et n est une valeur moyenne supérieure ou égale à 2 et de préférence comprise entre 4 et 50,

le nombre d'atomes de carbone dans chaque unité répétitive, identique ou différente, étant au moins égal à 4.

**7.** Utilisation selon la revendication 6, caractérisée par le fait que le polyéther oligomère est le diméthyléther du polytétrahydrofuranne répondant à la formule suivante :

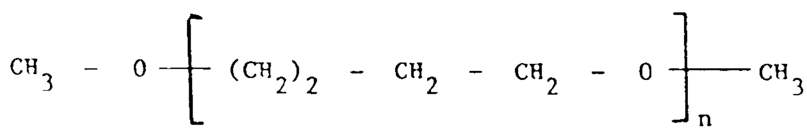

dans laquelle n est une valeur moyenne comprise entre 4 et 10.

**8.** Solution essentiellement anhydre de peroxyde d'hydrogène dans au moins un solvant organique caractérisée par le fait que ledit solvant est essentiellement constitué par un polyéther oligomère de formule :

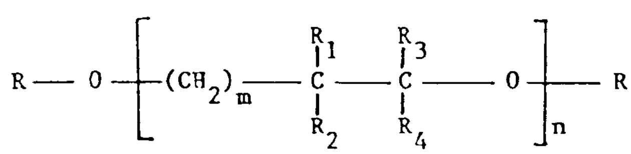

dans laquelle :

R représente un radical alkyle, linéaire ou ramifié, ayant de 1 à 12 atomes de carbone,

$R_1$, $R_2$, $R_3$ et $R_4$, identiques ou différents, représentent un atome d'hydrogène ou un radical alkyle ayant de 1 à 6 atomes de carbone, au moins deux des radicaux $R_1$, $R_2$, $R_3$ ou $R_4$ représentant un atome d'hydrogène,

m est 1 à 4,

et n est une valeur moyenne supérieure ou égale à 2 et de préférence comprise entre 4 et 50,

le nombre d'atomes de carbone dans chaque unité répétitive, identique ou différente, étant au moins égal à 4,

ladite solution contenant de 0,1 à 20 % en poids de peroxyde d'hydrogène, et moins de 1 % en poids d'eau.

**9.** Solution essentiellement anhydre selon la revendication 8, caractérisée par le fait que ledit polyéther oligomère est le diméthyléther du polytétrahydrofuranne de formule :

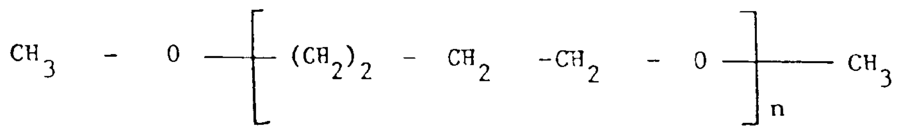

dans laquelle :

n est une valeur moyenne comprise entre 4 et 10,

ladite solution ayant une teneur en peroxyde d'hydrogène comprise entre 1 et 10 % en poids et une teneur en eau inférieure à 0,5 %.

## Claims

1. Use of an essentially anhydrous liquid solution of hydrogen peroxide, in at least one organic solvent, for the preparation of a therapeutic composition intended for the treatment of acne, dermatoses and skin ulcers, the said organic solvent being selected from an alcohol having from 2 to 20 carbon atoms, a polyol and an oligomeric polyether, and the said solution containing from 0.1 to 20% by weight of hydrogen peroxide as sole active principle and less than 1% by weight of water.

2. Use of an essentially anhydrous liquid solution of hydrogen peroxide, in at least one organic solvent, in operations of bleaching or oxidation dyeing of hair and of neutralisation of permanent waving or straightening of hair, the said organic solvent being selected from an alcohol having from 2 to 20 carbon atoms, a polyol and an oligomeric polyether, and the said solution containing from 0.1 to 20% by weight of hydrogen peroxide as sole active principle and less than 1% by weight of water.

3. Use according to one of Claims 1 or 2, characterised in that the essentially anhydrous solution has a hydrogen peroxide content of between 1 and 10% by weight and a water content of less than 0.5%.

4. Use according to one of Claims 1 or 2, characterised in that the alcohol having from 2 to 20 carbon atoms is selected from- the group consisting of ethanol, n-propanol, amyl alcohol, isopropanol, oleyl alcohol, cyclohexanol and benzyl alcohol.

5. Use according to one of Claims 1 or 2, characterised in that the polyol is selected from the group consisting of ethylene glycol, propylene glycol, diethylene glycol, glycerol and their ethers.

6. Use according to one of Claims 1 or 2, characterised in that the oligomeric polyether is selected from those of ethylene oxide, of propylene oxide and their ethers, as well as from the oligomeric polyethers corresponding to the following formula:

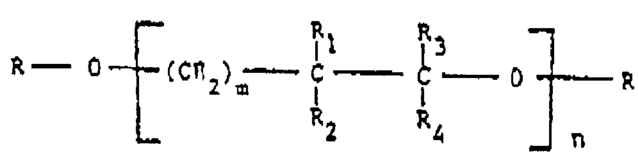

in which:

R denotes a linear or branched alkyl radical having from 1 to 12 atoms,

$R_1$, $R_2$, $R_3$ and $R_4$, which may be identical or different, denote a hydrogen atom, or an alkyl radical having from 1 to 6 carbon atoms, at least 2 of the radicals $R_1$, $R_2$, $R_3$ or $R_4$ denoting a hydrogen atom,

m is 1 to 4,

and n is an average value of not less than 2 and preferably between 4 and 50,

the number of carbon atoms in each identical or different repeated unit being at least equal to 4.

7. Use according to Claim 6, characterised in that the oligomeric polyether is polytetrahydrofuran dimethyl ether corresponding to the following formula:

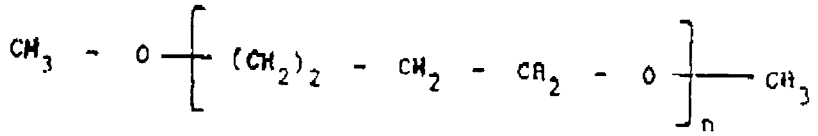

in which n is an average value of between 4 and 10.

8. Essentially anhydrous hydrogen peroxide solution in at least one organic solvent, characterised in that the said solvent essentially consists of an oligomeric polyether of formula:

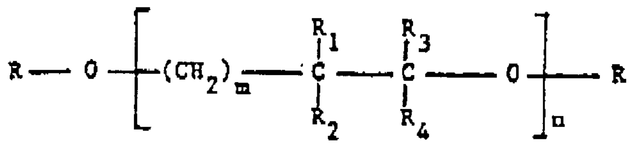

in which:

R denotes a linear or branched alkyl radical having from 1 to 12 carbon atoms,

$R_1$, $R_2$, $R_3$ and $R_4$, which may be identical or different, denote a hydrogen atom or an alkyl radical having from 1 to 6 carbon atoms, at least two of the radicals $R_1$, $R_2$, $R_3$ or $R_4$ denoting a hydrogen atom,

m is 1 to 4,

and n is an average value of not less than 2 and preferably between 4 and 50,

the number of carbon atoms in each identical or different repeated unit being at least equal to 4, the said solution containing from 0.1 to 20% by weight of hydrogen peroxide and less than 1% by weight of water.

9. Essentially anhydrous solution according to Claim 8, characterised in that the said oligomeric polyether is polytetrahydrofuran dimethyl ether of formula:

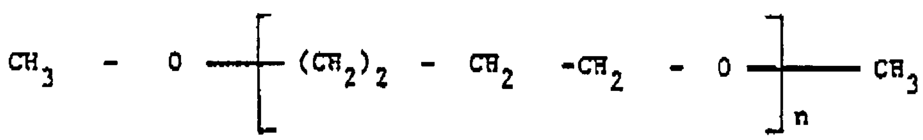

in which:

n is an average value of between 4 and 10, the said solution having a hydrogen peroxide content of between 1 and 10% by weight and a water content of less than 0.5%.

**Patentansprüche**

1. Verwendung einer flüssigen, im wesentlichen wasserfreien Lösung von Wasserstoffperoxid in mindestens einem organischen Lösungsmittel zur Herstellung einer therapeutischen Zusammensetzung, die zur Behandlung von Akne, Dermatosen und Hautgeschwüren bestimmt ist, wobei das organische Lösungsmittel unter Alkoholen mit 2 bis 20 Kohlenstoffatomen, Polyolen und oligomeren Polyethern ausgewählt ist und die Lösung 0,1 bis 20 Gew.-% Wasserstoffperoxid als einzigen Wirkstoff und weniger als 1 Gew.-% Wasser enthält.

2. Verwendung einer flüssigen, im wesentlichen wasserfreien Lösung von Wasserstoffperoxid in mindestens einem organischen Lösungsmittel bei der oxidativen Entfärbung oder Färbung der Haare und der Neutralisation von Dauerwellen oder dem Entkrausen der Haare, wobei das organische Lösungsmittel unter Alkoholen mit 2 bis 20 Kohlenstoffatomen, Polyolen und oligomeren Polyethern ausgewählt ist und die Lösung 0,1 bis 20 Gew.-% Wasserstoffperoxid als einzigen Wirkstoff und weniger als 1 Gew.-% Wasser enthält.

3. Verwendung nach Anspruch 1 oder 2, dadurch gekennzeichnet, daß die im wesentlichen wasserfreie Lösung einen Wasserstoffperoxidgehalt von 1 bis 10 Gew.-% und einen Wassergehalt unter 0,5 % aufweist.

4. Verwendung nach Anspruch 1 oder 2, dadurch gekennzeichnet, daß der Alkohol mit 2 bis 20 Kohlenstoffatomen unter Ethanol, n-Propanol, Amylalkohol, Isopropanol, Oleylalkohol, Cyclohexanol und Benzylalkohol ausgewählt ist.

5. Verwendung nach Anspruch 1 oder 2, dadurch gekennzeichnet, daß das Polyol unter Ethylenglycol, Propylenglycol, Diethylenglycol, Glycerin und deren Ethern ausgewählt ist.

6. Verwendung nach Anspruch 1 oder 2, dadurch gekennzeichnet, daß der oligomere Polyether ausgewählt ist unter den Polyethern aus Ethylenoxid, aus Propylenoxid und aus deren Ethern sowie den oligomeren Polyethern der Formel

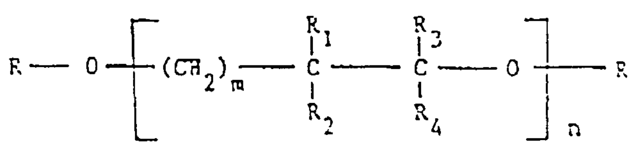 ,

in der bedeuten:

| | |
|---|---|
| R | geradkettiges oder verzweigtes $C_{1-12}$-Alkyl, |
| $R_1$, $R_2$, $R_3$ und $R_4$, | die gleich oder verschieden sein können, Wasserstoff oder $C_{1-6}$-Alkyl, wobei mindestens zwei der Gruppen $R_1$, $R_2$, $R_3$ und $R_4$ Wasserstoff sind, |
| m | 1 bis 4 und |
| n | einen mittleren Wert $\geqq 2$ und vorzugsweise von 4 bis 50, wobei die Anzahl der Kohlenstoffatome in jeder wiederkehrenden Einheit, die gleich oder unterschiedlich sein kann, mindestens gleich 4 ist. |

7. Verwendung nach Anspruch 6, dadurch gekennzeichnet, daß der oligomere Polyether der Dimethylether eines Polytetrahydrofurans der Formel

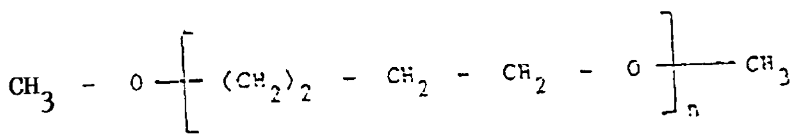

ist, in der n einen mittleren Wert von 4 bis 10 bedeutet.

8. Im wesentlichen wasserfreie Lösung von Wasserstoffperoxid in mindestens einem organischen Lösungsmittel, dadurch gekennzeichnet, daß das Lösungsmittel im wesentlichen aus einem oligomeren Polyether der Formel

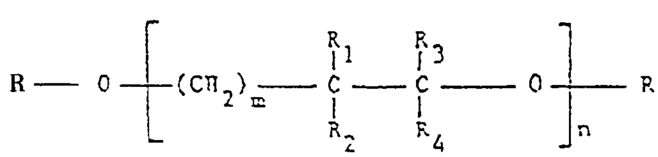

besteht, in der bedeuten:

| | |
|---|---|
| R | geradkettiges oder verzweigtes $C_{1-12}$-Alkyl, |
| $R_1$, $R_2$, $R_3$ und $R_4$, | die gleich oder verschieden sein können, Wasserstoff oder $C_{1-6}$-Alkyl, wobei mindestens zwei der Gruppen $R_1$, $R_2$, $R_3$ und $R_4$ Wasserstoff sind, |
| m | 1 bis 4 und |
| n | einen mittleren Wert $\geqq 2$ und vorzugsweise von 4 bis 50, |

wobei die Anzahl der Kohlenstoffatome in jeder wiederkehrenden Einheit, die gleich oder unterschiedlich

sein kann, mindestens gleich 4 ist und wobei die Lösung 0,1 bis 20 Gew.-% Wasserstoffperoxid und weniger als 1 Gew.-% Wasser enthält.

9. Im wesentlichen wasserfreie Lösung nach Anspruch 8, dadurch gekennzeichnet, daß der oligomere Polyether der Dimethylether eines Polytetrahydrofurans der Formel

$$CH_3 - O - \left[ (CH_2)_2 - CH_2 - CH_2 - O \right]_n - CH_3$$

ist, in der
n einen mittleren Wert von 4 bis 10 bedeutet,
wobei die Lösung einen Wasserstoffperoxidgehalt von 1 bis 10 Gew.-% und einen Wassergehalt unter 0,5 % aufweist.